# EUROPEAN PATENT APPLICATION

(11) **EP 4 483 790 A1**
(43) Date of publication of application: **01.01.2025**
(21) Application number: 23181414.6
(22) Date of filing: 26.06.2023
(51) Int. Cl.: A61B 5/02, A61B 5/021

(54) **SYSTEM FOR USE IN HEMODYNAMIC PARAMETER MEASUREMENT**

(71) Applicant: Koninklijke Philips N.V., 5656 AG Eindhoven (NL)
(72) Inventor: LOUWSMA, Hendrik Klaas, 5656AG Eindhoven (NL); YAU, Kam Hing, 5656AG Eindhoven (NL); TAMMINGA, Stephanus Jacob Gerardus, 5656AG Eindhoven (NL); VAN DE VEEN, Egbert, 5656AG Eindhoven (NL); WESSELS, Arnoldus Cornelis, 5656AG Eindhoven (NL); VAN DER VLIS, Peter Hans, 5656AG Eindhoven (NL); SCHIPPER, Niels, 5656AG Eindhoven (NL); DUINEVELD, Paulus Cornelis, 5656AG Eindhoven (NL); AMINE EL SAYED, Achraf, 5656AG Eindhoven (NL)
(74) Representative: Philips Intellectual Property & Standards

(57) **Abstract**

A system is provided for use in non-invasive hemodynamic parameter measurement. The system includes a pressure applicator or actuator for use in applying a variable pressure to a body part of a user. The system further comprises a shell structure for encircling the body part of the patient and being discontinuous tangentially so as to have at least two overlapping ends which can slide tangentially relative to one another. The system further includes a tissue pressure sensor for disposal between the shell structure and the body part during operation. A controller controls an applied pressure sequence in which the applied pressure is advanced incrementally through a series of discrete applied pressure values, the applied pressure being held constant at each applied pressure value, and the tissue pressure sensor signal being sampled during each of the applied pressure levels. Thus, a step-wise applied pressure cycle is implemented and wherein the tissue pressure is sampled during each static applied pressure step. This reduces stick-slip artefacts in the sampled tissue pressure signal arising from the tangential sliding of the overlapping surfaces of the shell structure.

## Description

### FIELD OF THE INVENTION

The present invention relates to the field of non-invasive hemodynamic parameter measurement.

### BACKGROUND OF THE INVENTION

The most common method for non-invasive blood pressure (NIBP) measurement is the use of an oscillometric blood pressure measurement cuff. The cuff comprises an inflatable bladder. The cuff wraps around the upper arm of a patient and is controlled to progress through an inflation and deflation cycle during which a pressure inside the bladder is sampled and a single set of values for systolic arterial pressure and diastolic arterial pressure are derived through processing of the pressure signal.

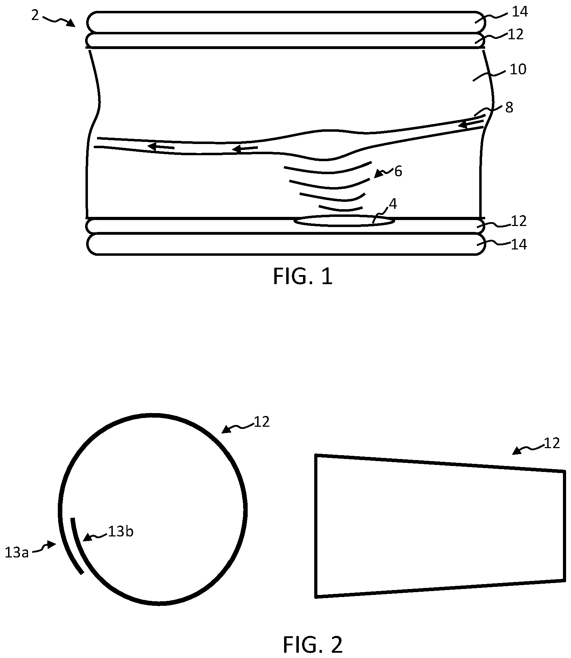

A variant approach to non-invasive blood pressure measurement has previously been proposed. This also uses a cuff which wraps around the body part of the patient. A cross-sectional illustration is shown in Fig. 1. The cuff 2 is shown wrapped around the arm 10 of a patient, and the brachial artery 8. The cuff 2 includes a pressure actuator 14, which optionally may be an inflatable bladder, as in the traditional NIBP cuff. In variance with the traditional NIBP cuff, the variant cuff also includes a separate tissue pressure sensor 4 arranged to measure a pressure between the body part 10 and the cuff. The tissue pressure sensor in some examples comprises a fluid-filled sensor pad, wherein a pressure in the pad varies as a function of pressure exerted on the pad. Also in variance with the traditional NIBP cuff, the variant cuff includes a hard shell 12 which extends around the body part and is disposed (radially) between the actuator 14 and the body part 10.

The pulse waves in the brachial artery 8 cause a change in the skin movement. This change in skin movement causes a compression of the liquid in the sensor pad 4. The sensor pad is fluidly connected via a flexible tube filled with similar liquid to a pressure transducer which measures the pressure signal.

The pressure sensor pad 8 is enclosed by a hard shell 12 to enable stiffness and enable a sufficient signal from the pressure sensor. In similarity with a standard NIBP cuff, the pressure actuator 14 which may comprise a bladder which is inflatable with an air pump, to compress the arm tissue, so that the brachial artery 8 can be closed.

Due to the inclusion of the separate tissue pressure sensor 4 with hard shell 12 backing, this cuff can obtain a real time pulse signal for the patient, and, from this, multiple different hemodynamic parameters can be obtained, including stroke volume and cardiac output.

The shell structure 12 has an annular form, and, in the most common design, is formed from a single piece of material which is curled round to span a complete (closed) loop. Fig. 2 schematically shows an example shell structure 12 according to the state of the art. Fig. 2 (left) shows a cross-section across a plane normal to a longitudinal axis of the shell and parallel with tangential and radial axes of the shell. Fig. 2 (right) shows a cross-section across a plane parallel with a longitudinal axis of the shell.

The shell structure is for extending around the body part 10 and is for arrangement between the tissue pressure sensor and the pressure actuator during operation, the shell structure being discontinuous circumferentially so as to having at least one pair of tangentially overlapping edge surfaces 13a, 13b which are tangentially slidable relative to one another during operation.

### SUMMARY OF THE INVENTION

It has been recognized by the inventors that the tangential sliding of the overlapping area of the shell structure can lead to artefacts in the measured signal. In particular, whenever two surfaces slide relative to one another, there is a tendency for occurrence of stick-slip. Stick slip is a type of intermittent motion that occurs when two surfaces slide relative to one another. It is characterized by alternating periods of "sticking" (static friction) and "slipping" (dynamic friction). This behavior arises due to the differences in the coefficients of static and kinetic friction between the two surfaces. When the two surfaces are at rest (in the "stick" phase), the static friction force between them is sufficient to prevent movement. As the external force applied to the surfaces increases, it eventually overcomes the static friction force, causing the surfaces to begin sliding against each other. This is the "slip" phase, in which the friction force acting on the surfaces is dynamic friction, which is typically lower than static friction.

Once the surfaces are in motion, the force required to maintain movement is generally less than the force needed to initiate it. This causes the surfaces to decelerate and eventually come to a stop again. At this point, the cycle repeats, and the surfaces enter another "stick" phase.

The inventors have recognized that the stick-slip action can lead to intermittent artefacts in the tissue pressure signal as the shell suddenly moves from stick to slip phase, leading to a non-physiological sudden fluctuation in the measured tissue pressure signal.

One way of reducing stick-slip is by carefully tuning the static and kinetic coefficients of friction of the two surfaces. However, while this can reduce the phenomenon, it cannot eliminate it entirely.

At least one aim of embodiments of the present invention is to further reduce the detrimental effect of stick-slip on pressure measurements.

The invention is defined by the claims.

According to examples in accordance with an aspect of the invention, there is provided a system for use in hemodynamic parameter measurement comprising: a cuff for extending around a body part of a user, and wherein the cuff includes a pressure actuator operable to apply a variable pressure to the body part by the cuff; a tissue pressure sensor arranged for sensing a pressure between a surface of the body part and the cuff; a shell structure for extending around the body part and for arrangement between the tissue pressure sensor and the pressure actuator during operation, the shell structure having at least one pair of tangentially overlapping surfaces which are tangentially slidable relative to one another to permit variation in a diameter of the shell structure; and a controller unit.

The controller unit is adapted to:
control a measurement cycle for the cuff which comprises controlling the pressure actuator to progress through a discrete sequence of applied pressure values, wherein each applied pressure value is held constant for an application period; and
record a respective tissue pressure signal from the tissue pressure sensor during each application period.

Thus, a sequence of discrete tissue pressure measurements is acquired, at each of a series of applied pressures which the pressure actuator is controlled to advance through in stepwise fashion. Each tissue pressure measurement may comprise a recorded set of one or more pressure pulses, each pressure pulse corresponding to one heart cycle. This measurement approach results in a discrete set of measurements, each associated with an applied pressure value, and each having a sampled tissue pressure signal recorded therefor.

Preferably, a transition time between each pair of consecutive discrete applied pressure values is shorter than any application period. This allows the measurement to be performed rapidly.

In some embodiments, the actuator comprises an inflatable bladder, wherein an inflation state of the bladder determines the applied pressure.

Preferably, the sequence of applied pressure values is an incrementing sequence of applied pressure values; in other words of sequence of increasing pressure values. In other words, the actuator is controlled to increase the applied pressure in a step-wise fashion. However, a decreasing sequence of applied pressure values is also an option.

There is thus proposed a system for hemodynamic parameter measurement in which the applied pressure is controlled in a stepped fashion. For example an inflation cycle of an inflatable bladder is controlled in a stepped fashion. This has the advantage of eliminating stick-slip artefacts associated with the contraction or expansion of the shell between the different applied pressure values. In particular, because the applied pressure is always held constant throughout the period where the tissue pressure is being sampled, this means that, even if stick-slip effects do occur, they occur in-between periods of sensor sampling, and thus do not impact the sensor measurements themselves.

The stepped applied pressure cycle effectively represents a reduction in the amount of sampled data which is used in computing hemodynamic parameter measurements, with some consequent loss in signal. However, it is the recognition of the inventors that the improved measurement quality achieved by elimination of the stick slip artefacts outweighs the loss of measurement signal. Thus, in the context of the measurement cuff having the shell structure with a sliding overlap, the implementation of a stepped applied pressure cycle has technical benefit.

In some embodiments, the method further comprises computing one or more hemodynamic parameters based on the set of tissue pressure measurements obtained for the sequence of discrete applied pressure values. For example, for each applied pressure level, a respective sub-set of tissue pressure measurement data may be acquired. Each sub-set of tissue pressure measurement data may comprise a recording of a set of one or more pressure pulses or cycles. Each pressure pulse corresponds to a heart wave cycle.

In some embodiments, the method may comprise recording tissue pressure sensor data continuously throughout the measurement cycle, and, once the measurement cycle is complete, processing the recorded sensor data and extracting a respective sub-set of tissue pressure data corresponding to each of the discrete applied pressure levels, i.e. corresponding to each application period. This may be based for example on identifying in the recorded continuous signal data signal segments for which the baseline pressure in the signal is flat (constant), these corresponding to periods of constant applied pressure, i.e. the measurement periods.

In some embodiments, the applied pressure values of the discrete sequence of applied pressure values may be pre-determined. Optionally, the applied pressure values are spaced at regular pressure intervals.

In some embodiments, at least a subset of the discrete sequence of applied pressure values are determined in real time during the measurement cycle based on acquired tissue pressure measurements.

In some embodiments, a transition between two applied pressure levels in the sequence may comprise: identifying or determining a target next applied pressure level in the sequence; transitioning the actuator applied pressure to the target applied pressure level while simultaneously sampling and recording the tissue pressure sensor signal; extracting a baseline trend from the tissue pressure signal over the transition period, determining an expected change in the baseline trend in view of the change in applied pressure; and comparing the actual change in the baseline trend over the transition period with the expected change. If the actual change is less than the expected change by a pre-determined threshold amount, the applied pressure may be slowly adjusted further along the cycle, e.g. slowly further increased, until the baseline trend rises or drops to a level expected, given the total change in applied pressure.

As discussed previously, a characteristic of the shell-backed cuff design is the potential for stick-slip effects between the overlapping surfaces of the shell structure during inflation and/or deflation. The above approach allows for accommodating instances in which the shell has become stuck and a change in actuator pressure has not yet caused the shell to adjust. The applied pressure can be further increased or decreased (as appropriate) until the shell moves, this being registered and detectable as a change in the baseline of the tissue pressure signal.

In some embodiments, for at least a subset of the sequence of applied pressure values, a next applied pressure value in the sequence may be determined based on a tissue pressure measurement obtained at a current or previous applied pressure value of the sequence.

In some embodiments, the controller unit is adapted to monitor an amplitude of the tissue pressure signal recorded at each applied pressure, detect occurrence of a peak in the amplitude of the tissue pressure signal, determine a termination pressure for the measurement cycle based on the applied pressure value coincident with the detected peak amplitude, and terminate the measurement cycle at said determined termination pressure.

The maximum amplitude tissue pressure signal is related to systolic arterial pressure (SAP). It is advantageous to terminate the inflation cycle once SAP has been reached, in order to minimize the total time duration of the measurement, for reasons of comfort of the patient and convenience of the caregiver.

The inventors have found that the SAP is expected to occur at an applied pressure value of corresponding to about 1.1-1.4 times the applied pressure value where the amplitude has reached its maximum (and in practice usually around 1.2 times). The exact multiplication factor may depend upon physiological parameters and is for example explained in detail in the document WO 2018/210931 A1.

Thus, in some embodiments, the termination pressure may be determined as a value between 1-1.3x the applied pressure value coincident with the detected peak amplitude in the tissue pressure signal.

In some embodiments, the controller unit is adapted to obtain an expected value for a diastolic arterial pressure (DAP) of the user, and determine a first applied pressure value of the sequence based on said expected value for diastolic arterial pressure (DAP). The DAP may be obtained from a previous measurement in some examples.

It has been found by the inventors that it may be optimal to start the applied pressure sequence at an applied pressure expected to coincide with a tissue pressure signal having an amplitude which is just below an expected value of the DAP, e.g. at 70-90% of the DAP. This advantageously minimizes the duration of the measurement duration by cutting out applied pressure values below the minimum arterial blood pressure of the user.

In some embodiments, each application period spans at least 1-3 heart pulses.

In some embodiments, each application period spans a single heart cycle. If each tissue pressure signal is sampled for just a single heart cycle for each applied pressure value, this minimizes the total time duration of the measurement cycle.

Furthermore, in this way it is possible to acquire many pulses during a single breathing cycle. This makes it possible to also obtain information on the heart lung interaction. The heart-lung interaction has an influence on the height of the tissue pressure pulse: it results in an oscillation superposed atop an envelope of the tissue pressure signal. This heart lung interaction is especially visible for sedated persons. Measurements might be obtained during the inflation cycle and/or the deflation slope.

In some embodiments, the cuff further comprises a releasable securement means for securing the wrapped cuff around the arm to prevent unwrapping, and wherein the securement means comprises a hook and loop type coupling. It is known that slipping of the hook-and-loop fastening of a blood pressure cuff is another source of signal artefact. These artefacts are known as cuff cracks. The proposed scheme of stepped pressure increase, with measurements obtained at each of a series of temporarily static applied pressure values also helps to minimize cuff crack artefacts, since cuff-cracks would typically only be expected to occur during period of variation of the applied pressure.

It is noted that the discrete sequence of applied pressure levels may be a sequence of increasing pressure levels or a sequence of decreasing pressure levels. In some embodiments, the sequence may run in both directions: first advancing through a series of increasing applied pressure steps before advancing through a sequence of decreasing applied pressure steps.

A further aspect of the invention provides a method for controlling a measurement cycle of a hemodynamic parameter measurement apparatus. The apparatus comprises: a cuff for wrapping around a body part of a user, and wherein the cuff includes a pressure actuator operable to apply a variable pressure to the body part by the cuff; a tissue pressure sensor arranged for sensing a pressure between a surface of the body part and the cuff; and a shell structure for extending around the body part and for arrangement between the tissue pressure sensor and the pressure actuator during operation, the shell structure having at least one pair of tangentially overlapping surfaces which are tangentially slidable relative to one another to permit variation in a diameter of the shell structure.

The method comprises:
controlling a measurement cycle for the cuff which comprises controlling the pressure actuator to progress through a discrete sequence of applied pressure values, wherein each applied pressure value is held constant for an application period; and
recording a respective tissue pressure signal from the tissue pressure sensor during each application period.

Optionally, a transition time between each pair of consecutive discrete pressure values is shorter than any application period.

The method is machine-implemented. The method may be computer implemented. The method may be implemented by control circuitry. The method is not a mental method.

The method may further comprise monitoring an amplitude of the tissue pressure signal recorded at each applied pressure, detecting occurrence of a peak in the amplitude of the tissue pressure signal, determining a termination pressure for the measurement cycle based on the applied pressure value coincident with the detected peak amplitude, and terminating the measurement cycle at said determined termination pressure.

The method may further comprise obtaining an expected value for a diastolic arterial pressure (DAP) of the user, and determining a first applied pressure value of the sequence based on said expected value for diastolic arterial pressure (DAP).

It has been found by the inventors that it may be optimal to start the applied pressure sequence at an applied pressure expected to coincide with a tissue pressure signal having an amplitude which is just below an expected value of the DAP, e.g. at 70-90% of the DAP. This advantageously minimizes the duration of the measurement by cutting out applied pressure values below the minimum arterial blood pressure of the user.

Another aspect of the invention is a computer program product comprising code means configured, when run on a processor operatively coupled with a hemodynamic parameter measurement in accordance with any example or embodiment described in this document, to cause the processor to perform a method in accordance with any example of embodiment described in this document.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example apparatus suitable for use as part of embodiments of the present invention;
Fig. 2 shows schematic representations of a shell portion of the apparatus of Fig. 1;
Fig. 3 is a block diagram of an example system in accordance with one or more embodiments of the invention;
Fig. 4 outlines steps of an example method in accordance with one or more embodiments of the invention;
Fig. 5 illustrates a tissue pressure signal recorded over a continuously increasing applied pressure ramp;
Fig. 6 illustrates example stick-slip artefacts in recorded tissue pressure signals;
Fig. 7 illustrates an example tissue pressure signal recorded over a continuously increasing applied pressure ramp;
Fig. 8 illustrates the principle of computing hemodynamic parameters using tissue pressure signals recorded at only a selected discrete set of applied pressure levels; Fig. 9 illustrates example envelope curves from two consecutive measurement cycles implemented using a discrete measurement cycle in accordance with embodiments of the present invention;
Fig. 10 illustrates a set of tissue pressure signals acquired at each of a sequence of discrete applied pressure values in accordance with one or more embodiments of the present invention;
Fig. 11 illustrates theoretical maximum inflation rates for two example blood pressure measurement cuffs, illustrating the capacity for rapid transition between two subsequent applied pressure levels; and
Fig. 12 schematically illustrates one example applied pressure control approach in which the discrete applied pressure levels are dynamically configured in real time to account for potential stick-slip effects.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for use in non-invasive hemodynamic parameter measurement. The system includes a pressure applicator or actuator for use in applying a variable pressure to a body part of a user. The system further comprises a shell structure for encircling the body part of the patient and being discontinuous tangentially so as to have at least two overlapping ends which can slide tangentially relative to one another. The system further includes a tissue pressure sensor for disposal between the shell structure and the body part during operation. A controller controls an applied pressure sequence in which the applied pressure is advanced through a series of discrete applied pressure values, the applied pressure being held constant at each applied pressure value, and the tissue pressure sensor signal being sampled during each of the applied pressure levels. Thus, a step-wise applied pressure cycle is implemented and wherein only the tissue pressure signal segments sampled during each static applied pressure step are used in computing hemodynamic parameters. This reduces stick-slip artefacts in the sampled tissue pressure signal arising from the tangential sliding of the overlapping surfaces of the shell structure.

By way of background, Fig. 1 shows a schematic illustration of the basic layered structure of a prior art hemodynamic parameter measurement apparatus of which embodiments of the present invention represent a development.

The apparatus can be used as part of a system or method for detecting blood pressure and/or other hemodynamic parameter measurements such as cardiac output or stroke volume. The illustration shows the cuff 2 applied to the upper arm 10 of a patient. An artery 8 of the patient is schematically shown. The apparatus has a design which differs from most standard cuff-based blood pressure measurement apparatuses in that it includes a dedicated tissue pressure sensor 4 which the cuff acts to hold against the tissue of the skin when the cuff is inflated. This feature is shared by embodiments of the present invention.

The cuff 2 includes a pneumatic pressure actuator 14 in the form of an inflatable bladder for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled. Instead of a pneumatic actuator, any other form of pressure actuator can be used.

The cuff 2 further includes a shell structure 12, wherein the shell structure is arranged so as to be located between the pneumatic actuator 14 and the body part 10 when the cuff is worn on the body part, and arranged to surround the body part when the cuff is worn. The shell structure may be relatively stiff. Consequently, the measurement accuracy can be improved, since the pressure in the arm is measured by the tissue pressure sensor pad 4 against a relatively stiff support, which prevents dampening of the amplitude and shape of the signal. In particular, if the tissue pressure sensor pad 4 is located at least partially between the shell structure 12 and the body part 10, a high accuracy of the signals measured by the tissue pressure sensor unit can be achieved. With such a configuration of the tissue pressure sensor pad 4, the shell structure 12 does not absorb or attenuate the arterial pressure signal.

The apparatus of Fig. 1 differs from standard blood pressure measurement cuffs. Standard cuffs use the bladder air pressure as the sole measurement signal for sensing blood pressure. By additionally or alternatively utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g. a fluid-filled bag which is fluidically coupled to a pressure transducer for read-out of a tissue pressure signal. When blood pulses in the artery 8, this results in a pressure wave 6 which can be detected by the tissue pressure sensor 4. The tissue pressure sensor 4 can be connected to the pressure transducer via a fluid-filled tube/line. The pressure transducer converts the pressure in the fluid into an electrical signal.

Thus, the operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated actuator with rising clamping pressure. However, the actual compression may be achieved via the narrowing diameter of the rigid circumferential shell 12.

The cuff 2 may further comprise a releasable securement means for securing the wrapped cuff around the arm to prevent unwrapping. In some examples, the securement means may comprise a hook and loop type coupling (i.e. Velcro^{®}).

The shell structure 12 has an annular form, and, in the most common design, is formed from a single piece of material which is curled round to span a complete (closed) loop. Fig. 2 schematically shows an example shell structure 12 according to the state of the art. Fig. 2 (left) shows a cross-section across a plane normal to a longitudinal axis of the shell and parallel with tangential and radial axes of the shell. Fig. 2 (right) shows a cross-section across a plane parallel with a longitudinal axis of the shell.

The shell structure is for extending around the body part 10 and is for arrangement between the tissue pressure sensor and the pressure actuator during operation, the shell structure being discontinuous circumferentially so as to having at least one pair of tangentially overlapping edge surfaces 13a, 13b which are tangentially slidable relative to one another during operation.

Embodiments of the present invention represent a development of a shell-backed cuff, of which the above-outlined device represents an example. In particular, embodiments of the present invention provide a system and method with an improved control scheme for the applied pressure exerted by the actuator over the course of a measurement.

One aspect of the invention provides a system for use in hemodynamic parameter measurement.

The system comprises a cuff for extending around a body part of a user, and wherein the cuff includes a pressure actuator operable to apply a variable pressure to the body part by the cuff. The system further comprises a tissue pressure sensor arranged for sensing a pressure between a surface of the body part and the cuff. The system further comprises a shell structure for extending around the body part and for arrangement between the tissue pressure sensor and the pressure actuator during operation, the shell structure having at least one pair of tangentially overlapping surfaces which are tangentially slidable relative to one another. By way of illustrative example, these components of the system may be provided in the form of the device described above with reference to Fig. 1 and Fig. 2 including the cuff 2 with pressure actuator 14, shell structure 12 and tissue pressure sensor 4.

In addition, the system further includes a controller unit, adapted to perform a control method comprising: controlling a measurement cycle for the cuff which comprises controlling the pressure actuator to progress through a discrete sequence of applied pressure values, wherein each applied pressure value is held constant for an application period. Preferably, a transition time between each pair of subsequent discrete pressure values is shorter than any application period. The control method further comprises recording a respective tissue pressure measurement from the tissue pressure sensor during each application period.

Each tissue pressure measurement may comprise a recorded set of one or more pressure pulses, each pressure pulse corresponding to one heart cycle.

In some embodiments, the method further comprises computing one or more hemodynamic parameters based on the set of tissue pressure measurements obtained for the sequence of discrete applied pressure values. For example, for each applied pressure level, a respective sub-set of tissue pressure measurement data may be acquired. Each sub-set of tissue pressure measurement data may comprise a recording of a set of one or more pressure pulses or cycles. Each pressure pulse for example corresponds to a heart wave cycle.

In some embodiments, the method may comprise recording tissue pressure sensor data continuously throughout the measurement cycle, and, once the measurement cycle is complete, processing the recorded sensor data and extracting a respective sub-set of tissue pressure data corresponding to each of the discrete applied pressure levels, i.e. corresponding to each application period. This may be based for example on identifying in the recorded continuous signal data signal segments for which the baseline pressure in the signal is flat (constant), these corresponding to periods of constant applied pressure, i.e. the measurement periods.

The system represents one aspect of the invention. The control method implemented by the controller unit represents a separate aspect of the invention. The controller unit configured for implementing the control method represents another aspect of the invention.

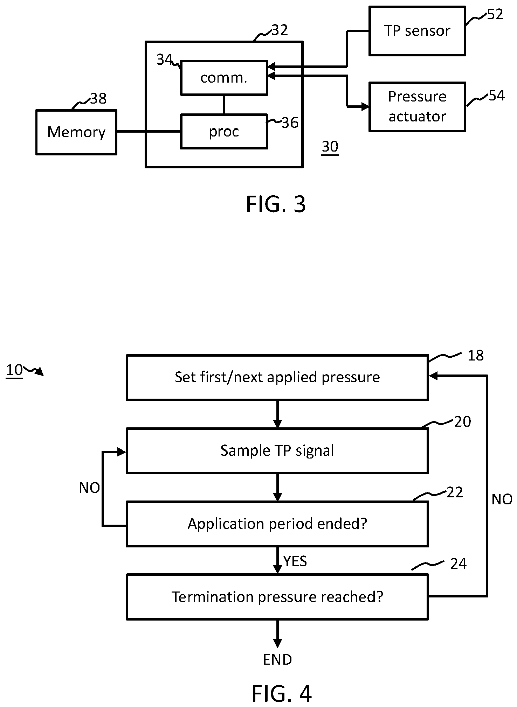

Fig. 3 shows a block diagram of the electronic components of an example system 30 in accordance with one or more embodiments.

A controller unit 32 is provided for controlling the applied pressure level of the pressure actuator 54 of the cuff in the manner discussed above. The tissue pressure ("TP") sensor 52 outputs a tissue pressure signal to the controller unit. In this illustrated example, the controller unit comprises one or more processors 36 for performing the above-mentioned control method. The controller unit further comprises an input/output 34 or communication interface for interfacing with the tissue pressure sensor 52 and the pressure actuator 54. The system 30 may further comprise a memory 38 for storing computer program code (i.e. computer-executable code) which is configured for causing the one or more processors 36 of the processing unit 32 to perform the control method as outlined above, or in accordance with any embodiment described in this disclosure, or in accordance with any claim.

The invention can also be embodied in software form. Thus another aspect of the invention is a computer program product comprising computer program code configured, when run on a processor, to cause the processor to perform the above-outlined control method or a control method in accordance with any example or embodiment of the invention described in this document, or in accordance with any claim of this patent application.

Fig. 4 outlines with greater specificity steps of one example method for controlling the applied pressure sequence of the pressure actuator in accordance with one or more embodiments.

The method 10 comprises setting 18 a first applied pressure value of the sequence of pressure values. As will be explained later below, in some embodiments, the method may comprise a step of determining a first applied pressure value of the sequence of applied pressure values. In some embodiments, this may be determined based on an expected value for diastolic arterial pressure (DAP). For example, in some embodiments, the starting applied pressure may be set at a value expected to coincide with a tissue pressure signal having an amplitude which is at a value 70-90% of the expected DAP. The expected DAP may be a pre-determined value, for example retrieved from a memory. It may be based on a previous measurement for the patient, or based on population values.

Holding the first applied pressure value constant, the tissue pressure sensor 52 signal is sampled 20 and recorded. The applied pressure value is held constant for a pre-determined application period, T. The method may comprise recurrently checking 22 whether the application period has expired, and, if not, continuing to sample the tissue pressure signal.

Once the application period has ended, the method may comprise determining 24 whether the current applied pressure value of the actuator is equal to or greater than a defined termination pressure. The termination pressure may be pre-determined in advance, for example based on an expected value for the systolic arterial pressure (SAP) for the patient. As will be explained further below, in some embodiments, the termination pressure may be determined and/or updated in real time during the method, for example based on the tissue pressure sensor readings. For example, in some embodiments, the method may include monitoring an amplitude of the tissue pressure signal recorded at each applied pressure, detecting occurrence of a peak in the amplitude of the tissue pressure signal, and determining a termination pressure for the measurement cycle based on the applied pressure value coincident with the detected peak amplitude. The maximum amplitude tissue pressure signal is related to systolic arterial pressure (SAP). It is advantageous to terminate the inflation cycle once SAP has been reached since this minimizes the total duration of the measurement cycle. The SAP is expected to occur at an applied pressure value corresponding to about 1.1-1.3 times the applied pressure value where the amplitude has reached its maximum.

If the termination pressure has not yet been reached, the method cycles back to the first step 18 and comprises setting a next discrete applied pressure value for the pressure actuator. This comprises controlling the actuator to transition from the previous applied pressure value to the next applied pressure value.

The same sequence of steps are then repeated for the new applied pressure value.

The steps of the control method 10 are cycled in a loop until it is determined 24 that the termination pressure has been reached, at which point the measurement cycle ends. At this point, preferably the applied pressure is reduced down to a minimum value, e.g. zero, to minimize discomfort for the patient.

The termination pressure may be determined iteratively during the measurement cycle in some embodiments. For example, as described above, the termination pressure may be set at a pressure related to the applied pressure coinciding with a peak in the tissue pressure signal amplitude. This peak in the tissue pressure signal amplitude can be determined or identified in an iterative manner. For example, the controller continues increasing the applied pressure in discrete increments, determines the tissue pressure amplitude at each applied pressure step, and compares it against the amplitude measured for the previous applied pressure step. If higher, a further applied pressure increment is implemented, and another tissue pressure signal sampled. If lower, then the controller determines that the tissue pressure signal amplitude measured at the immediately preceding applied pressure level was the maximum tissue pressure amplitude. In other words, the controller iteratively increases the applied pressure until tissue pressure amplitude is detected to drop, whereupon the immediately preceding applied pressure value is taken to be one coincident with peak tissue pressure amplitude. As discussed above, this tissue pressure amplitude can be related, via a pre-determined multiplier to a measure of the user's systolic arterial pressure.

To help explain the concepts underlying the invention as well as features of preferred embodiments, there will now be outlined a more detailed discussion of the problem which embodiments of the present invention aim to address, and a discussion of advantageous features implemented in accordance with one or more embodiments for solving this problem. For purposes of illustration, discussions below refer to a cuff employing a pressure actuator in the form of an inflatable bladder, and wherein a measurement cycle involves an inflation cycle. However, it is to be understood that the same principles apply equally for cuffs with a pressure actuator of any other form: all that is needed is a means for applying a controllable pressure to the body part of the user, and in particular to an artery in the body part of the user.

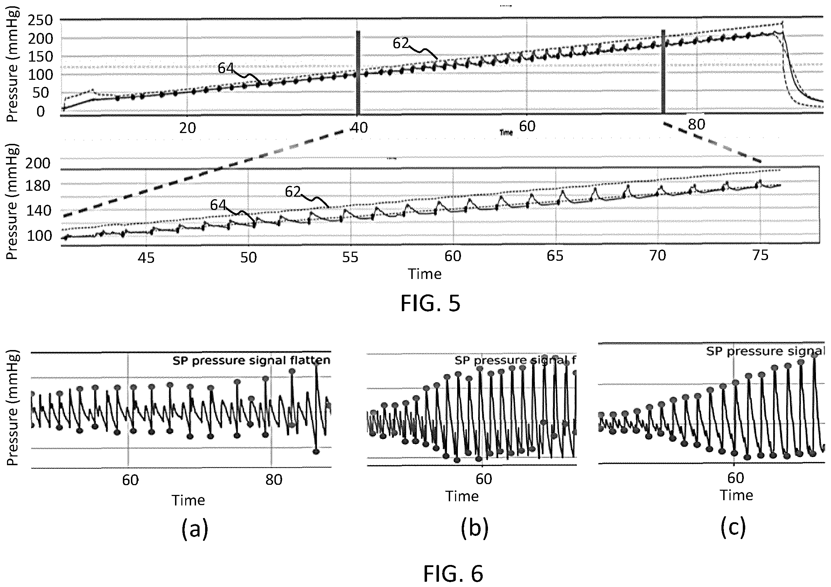

In known blood pressure measurement systems, an inflatable cuff is inflated with a continuously rising pressure, i.e. a continuous inflation ramp. A typical blood pressure (BP) measurement may typically take 1.5 minutes (90 seconds). Fig. 5 for example shows a plot of applied pressure (line 62) and measured tissue pressure (line 64) as a function of time over the course of a blood pressure measurement.

The upper plot shows the entire recording. The lower plot shows a zoomed view of a most relevant part of the signal.

It will be recognized that the tissue pressure signal 64 provides an effectively continuous signal output, a shape of which reflects a shape of a pulse wave signal for the patient.

From this obtained signal, blood pressure metrics such as values for MAP, SAP, and DAP are obtainable, as well as other hemodynamic parameters such as stroke volume (SV) and cardiac output (CO). In addition, heart rate can be read instantly based on the tissue pressure signal or a processed version thereof.

Suitable algorithms for processing the tissue pressure to derive the above-described measurements can be found for example in any of the documents: WO 2018/210931, WO 2020/148137, WO 2019/211210, US10485432, EP2759258B1, and US10349849.

For example, and as described in WO 2018/210931, in some embodiments, values for SAP, DBP and MAP can be obtained based on identifying a peak in the amplitude of a series of measured tissue pressure pulses. The various blood pressure measures can then be determined based on identifying amplitude values of the tissue pressure pulse sequence at pre-defined reduced proportions of the peak amplitude value. The reader is referred to WO 2018/210931 for more details.

It has been recognized by the inventors that a problem encountered in state of the art systems is that the tangential sliding of the overlapping area of the shell structure 12 can lead to artefacts in the measured signal. In particular, whenever two surfaces slide relative to one another, there is a tendency for the occurrence of stick-slip effects. Stick slip is a type of intermittent motion that occurs when two surfaces slide relative to one another. It is characterized by alternating periods of "sticking" (static friction) and "slipping" (dynamic friction). This behavior arises due to the differences in the coefficients of static and dynamic friction between the two surfaces.

The effect of stick-slip on a recorded tissue pressure signal during an inflation cycle is illustrated in Fig. 6. The effect results in artefacts in the signals in the form of steep upward steps at locations between pulses. As the shell slips, it tightens itself around the arm, resulting in a rapid pressure increase at the sensor pad.

Fig. 6(a) shows the most severe stick slip artefacts, followed by Fig. 6(b) with less severe stick-slip artefacts, followed by Fig. 6(c) with the least severe stick-slip artefacts. The size of the artefacts in the plot of Fig. 6(a) are so large that the computation algorithm counts them as additional heartbeats. Consequently, the heartbeat shown on the monitor would be about double the true heart rate value. However, the smaller disturbances visible in Fig. 6(b) and Fig. 6(c) still may affect the calculation of the hemodynamic parameters as the shape of the individual heart pulses is deformed compared to an artefact-free signal.

It is possible to use artefact reduction algorithms to partially filter out such artefacts. However, such algorithms also have a risk of altering or deforming the underlying physiological signal component, and thus impacting signal reliability.

It is the realization of the inventors that these stick-slip artefacts can be almost entirely eliminated by altering the behavior of the pressure actuator during measurement. In particular, it is proposed to step through a sequence of discrete applied pressure levels, each one being held constant for a short period while the tissue pressure signal is sampled. This results in a set of tissue pressure signals, each corresponding to one of the discrete applied pressure values.

A clear benefit of this discrete method is that the shell does not move during the recording of the heartbeat pulses. Hence, stick slip artefacts will not be present in the discrete recordings.

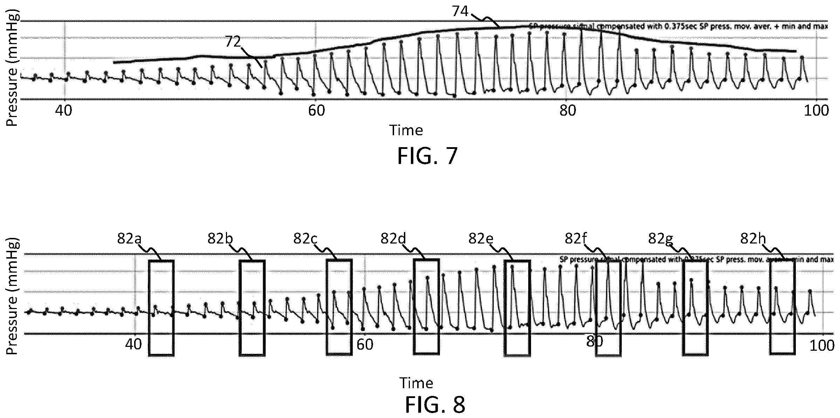

The principle is further illustrated in Fig. 7 and Fig. 8.

Fig. 7 shows a recorded tissue pressure signal 72 after subtraction of the baseline (meaning a trendline of the average of the signal). This has a shape which is indicative of the heartbeat signal of the patient. Line 74 shows an envelope of the tissue pressure signal which reflects a plot of the peak or maximal values of the tissue pressure signal as a function of applied pressure. It connects the peaks of the tissue pressure signal. It is indicative of a variation in the amplitude of the tissue pressure signal as a function of time. The location of the maximum of the envelope curve 74 is an important measure for determining when an SAP of the patient has been reached.

Fig. 8 schematically illustrates the measurement principle according to embodiments of the present invention. In particular, instead of deriving hemodynamic parameters based on a tissue pressure signal continuously recorded throughout a constant linear rise/ramp in applied pressure, it is proposed to record the tissue pressure signal for use in computing hemodynamic parameters only during a sequence of discrete pressure levels. It has been found by the inventors that when replacing the pressure ramp measurement method by a discrete applied pressure measurement method, similar hemodynamic parameters may still be obtained.

Fig. 8 illustrates respective tissue pressure signal portions of the signal of Fig. 7 during each of an example set of eight discrete applied pressure levels 82a-82h. Each tissue pressure signal portion in this example spans 2-3 heartbeat pulses. However, at minimum, one tissue pressure signal portion is sufficient. By connecting the eight maxima of these discrete tissue pressure signal portions, it is possible to obtain a similar envelope curve as compared to the continuous measurement method (illustrated in Fig. 7).

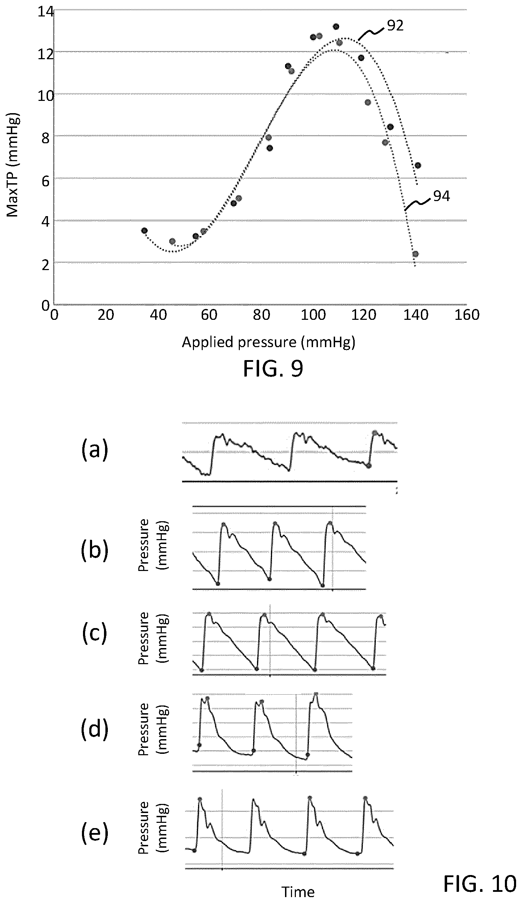

To illustrate this, Fig. 9 shows envelope curves extracted from two measurement cycles implemented in close temporal proximity of one another, each in accordance with the discrete measurement approach of the present invention. As mentioned above, an envelope curve of a tissue pressure signal reflects a plot of the peak or maximal values of the tissue pressure signal as a function of applied pressure. Line 92 shows the envelope curve of the first measurement cycle, and line 94 shows the envelope curve of the second measurement cycle performed shortly after the first. It can be seen that the peaks of these curves are very close together, and within the accuracy range in which one can determine the SAP value. The applied pressure value coinciding with the peak of the envelope curve is closely related to the SAP.

Other hemodynamic parameters can also be extracted from the envelope curve, including diastolic blood pressure (DBP) and mean arterial pressure (MAP). Methods for extracting these and other hemodynamic parameters from tissue pressure signal samples can be found in the following documents: WO 2018/210931, WO 2020/148137, WO 2019/211210, US10485432, EP2759258B1, and US10349849.

Existing algorithms for extracting hemodynamic parameters from the tissue pressure signals can be applied to the more reduced data set without major modification. For example, as illustrated in Fig. 9, an envelope signal can be obtained from the reduced set of tissue pressure signal pulses in a similar way as for a complete set of tissue pressure signal pulses, and parameters can be extracted from the envelope signal. In particular, the envelope signal represents the amplitudes of the sequence of tissue pressure signal pulses, and, as described in the above-referenced documents, a peak amplitude (a peak value of the envelope signal) can be used to compute values of SAP, DBP and MAP.

Thus, it is proposed in accordance with embodiments of the present invention to increase the applied pressure at a fast rate between each of a series of discrete pressure steps over a pressure range of interest. Thus, instead of increasing the applied pressure at a constant linear rate over time, it is proposed to instead increase the applied pressure step-wise.

In some embodiments, it is proposed to increase the applied pressure quickly to a value just below a value expected to coincide with a measured tissue pressure signal having an amplitude value equal to a diastolic blood pressure (DBP) for the patient. This may form a first applied pressure value of the sequence of applied pressure values.

In some embodiments, the sequence of applied pressure values may be values spaced at regular pressure intervals, e.g. 20 mmHg. In other words, the measurement cycle comprises increasing the applied pressure in steps or jumps of e.g., 20mmHg per step.

As discussed above, this approach still allows for deriving all relevant hemodynamic parameters accurately.

Fig. 10 illustrates a set of tissue pressure signals obtained at each of a discrete sequence of constant applied pressure levels. One or more hemodynamic parameter measurements are obtained from the set of tissue pressure signals.

The first signal (Fig. 10a) is recorded at an applied pressure of around 40 mmHg. This is well below the diastolic pressure of the subject and thus the pulse wave signal of the patient is only weakly represented in the measured signal. Presence of ripples and noise can be observed in the signal. However, the applied pressure value at which the first appearance of recognizable heartbeat pulses appears is also a relevant parameter for the algorithm that derives the hemodynamic parameters from the signal.

The second signal (Fig. 10b) is recorded at an applied pressure of around 77 mmHg, which is just below the diastolic pressure of the patient. Here, the noise in the signal has disappeared, and the signal is stronger and with a larger amplitude. For example, there is significantly improved visibility of the dicrotic notch, just below the maximum of the heartbeat pulse. However, below the dicrotic notch there is still some high frequency oscillation/fluctuation in the signal.

The third signal (Fig. 10c) is recorded at an applied pressure of around 87 mmHg. The amplitude has increased further. The waviness of the signal has entirely disappeared, and the pulses have a strong triangular shape. The minimum pressure of tissue pressure signal pulses is approximately equal the diastolic BP of 87 mmHg.

The fourth signal (Fig. 10d), is recorded at an applied pressure of around 98 mmHg. This is above diastolic pressure. The waveform begins to become distorted, since the artery is being closed by the higher cuff pressure. At the peaks of the pulses, there are some peaks with split tops. The amplitude of the pulses is still increasing.

The fifth signal (Fig. 10e) is recorded at an applied pressure of 110 mmHg. Here, the artery is fully closed. The amplitude of the pulses is at its largest, at 13mmHg. Here, the tissue pressure is maximum. Adding this amplitude to the baseline applied pressure results in a value for the systolic arterial pressure (SAP) of 123mmHg.

It may be observed that in none of the recorded tissue pressure signals are stick-slip artefacts present. Due to the maintenance of constant applied pressure during each respective recording, the overlapping area of the shell structure does not slide tangentially, as would otherwise be the case in a continuous inflation method. As a result, fewer artefacts overall are present in the recorded signal. This results in more accurate hemodynamic parameters derived from the tissue pressure signals.

Furthermore, using the discrete measurement approach, faster measurements are possible because fewer measurement points are required, and the actuator can be rapidly transitioned from one measurement point to the next.

Considering the continuous measurement approach, one way of achieving increases in measurement speed might be to simply increase the rate of applied pressure change over the measurement cycle, i.e. increasing the gradient of the applied pressure ramp. However, this would impact measurement accuracy. This is because, during the time spanned by a single pressure pulse during the existing (slow) continuous inflation method, the applied pressure changes only 2-3 mm Hg, and a correction for this effect is possible. However, if the inflation rate were increased further, e.g. to 7-8 mm Hg/s, this results in a much larger change in applied pressure over a single pressure pulse, with a consequently much more pronounced influence on the measurement. Compensating for this can only be done with more significant impact on the strength of the underlying physiological signal. The advantage of the method proposed herein is that the applied pressure remains constant during each individual discrete measurement. This allows for increasing the speed of the method without impacting on accuracy.

In some embodiments, it is proposed to control the applied pressure by increasing the applied pressure to a series of predefined discrete (and constant) levels to obtain the relevant hemodynamic parameters.

According to a further set of embodiments, it is proposed to determine at least a subset of the discrete applied pressure levels in real time during a measurement cycle based on acquired tissue pressure measurements.

In particular, in some embodiments, an iterative measurement method is proposed.

As discussed, an advantage of the pressure control method proposed by embodiments of the present invention is the capability to reduce the total time duration required for a measurement cycle by implementing rapid transitions between consecutive pressure steps. For example, in the case where the pressure actuator uses an inflatable bladder, inflation rates can be high, allowing for such rapid transitions.

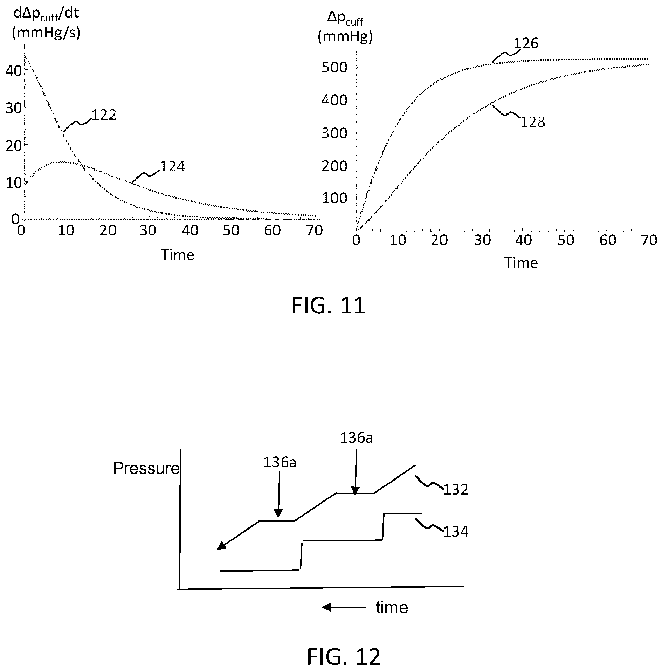

For example, Fig. 11 (left) shows a plot of a theoretical maximum inflation rate as a function of time for two example cuffs in current use. Line 122 shows the maximum inflation rate as a function of time for a first cuff and line 124 shows the maximum inflation rate as a function of time for a second cuff. The first cuff is of a smaller size to the second and so has a smaller total fluid volume than the second cuff. The total fluid volume of the smaller cuff is approximately 0.15-0.30 liters. The total fluid volume of the larger cuff is approximately 0.5-0.7 liters. The line shows the maximum inflation rate, meaning inflation rate at a maximum pump setting. Fig. 11 (right) shows the corresponding applied cuff pressure as a function of time, with line 126 corresponding to the first cuff and line 128 corresponding to the second cuff.

As demonstrated, at a cuff pressure of about 250 mmHg, the first cuff is capable of inflation rates of more than 25 mmHg/s and the second cuff is capable of inflation rates of more than 12.5 mmHg/s.

Taking the example case in which the actuator is controlled to increment through a sequence of applied pressure levels each being 20 mmHg apart, based on the illustrated inflation capacities, the smaller cuff can easily transition between consecutive measurement points in a second or less, i.e. approximately only the period of a single heartbeat. The larger cuff may take a little longer, e.g., 2-3 seconds to transition between measurement points. However, it will be recognized that, considering a sequence of e.g., 5-6 measurement points in total, in either case, this allows for a very rapid measurement cycle, particularly if each pressure level is only held constant for e.g., 1-3 heartbeats. For example, for the smaller cuff, a total measurement duration of less than 15 seconds is possible, while with the larger measurement cuff, a total measurement duration of around 20-30 seconds is possible.

This contrasts with a standard linearly ramping applied pressure cycle in which a total measurement duration may typically be between 60-90 seconds.

These durations can be further shorted by using the intelligent pressure level selection approaches mentioned previously. For example, the starting pressure level can be selected based on an estimated level for the DAP, and the measurement cycle can be terminated at or soon after detection of a maximum arterial pressure (SAP), based on detecting a peak amplitude in the tissue pressure signal. Upon reaching the SAP, the applied pressure escalation may be terminated and the applied pressure dropped to a lower, e.g., minimum value, e.g., zero. For example, the inflation may be stopped and the cuff immediately deflated.

As discussed previously, a characteristic of the shell-backed cuff design is the potential for stick-slip effects between the overlapping surfaces of the shell structure during inflation and/or deflation. It is the further recognition of the inventors that if a pre-determined sequence of applied pressure levels is used, in some cases, a transition between two pressure levels in the sequence may coincide with a 'stick' phase of the shell, meaning that the change in pressure of the actuator is not transferred onto the tissue, since the friction of the shells is preventing this from happening.

Thus, according to one or more embodiments, it is proposed that at least a subset of the applied pressure levels of the sequence of applied pressure levels be determined or adjusted in real time based on tissue pressure measurements. In particular, transition between two applied pressure levels in the sequence may comprise: identifying or determining a target next applied pressure level in the sequence, transitioning the actuator applied pressure to the target applied pressure level while simultaneously sampling and recording the tissue pressure sensor signal, extracting a baseline trend from the tissue pressure signal over the transition period, determining an expected change in the baseline trend in view of the change in applied pressure, and comparing the actual change in the baseline trend over the transition period with the expected change. If the actual change is less than the expected change by a pre-determined threshold amount, the applied pressure may be slowly adjusted further along the cycle, e.g. slowly further increased, until the baseline trend changes to a level expected, given the total change in applied pressure.

This approach allows for accommodating instances in which the shell has become stuck and a change in actuator pressure has not yet caused the shell to adjust. The applied pressure can be further increased or decreased until the shell moves, this being registered and detectable as a change in the baseline of the tissue pressure signal.

It is noted that this measurement approach can be applied for a measurement cycle in which the sequence of applied pressure values is an increasing sequence or decreasing sequence (e.g., during inflation or deflation). The sequence may run in both directions in some examples, first advancing through a series of increasing applied pressure steps before advancing through a sequence of decreasing applied pressure steps. Stick-slip is a more common phenomenon during decreasing applied pressure.

By way of illustration, Fig. 12 schematically illustrates an example deflation cycle of an actuator comprising an inflatable bladder controlled in accordance with the above-described procedure and wherein the shell portion is exhibiting stick-slip behavior. Line 132 shows the applied pressure level of the actuator. Line 134 represents an extracted baseline of the tissue pressure sensor. In other words, line 134 effectively represents the actual applied pressure experienced by the body part. Due to the stick-slip effect, the actual experienced applied pressure changes in discrete jumps, wherein, during each plateau, the shell is in a stick phase, and wherein each jump corresponds to a slip phase.

Arrows 136a and 136b represent measurement points for the measurement cycle, i.e. points at which the tissue pressure signal is sampled and recorded for use in subsequent computation of one or more hemodynamic parameters.

It can be seen that, due to the friction of the shell structure, the pressure experienced by the tissue (line 134) will remain constant for some time until the actuator pressure 132 has dropped sufficiently, and the shell starts to move again, and the tissue pressure drops. By recording the actuator pressure and the tissue pressure, the actuator can be stopped when the tissue pressure has dropped. Note that, each time the actuator pressure stops, the shells will stop moving and the actuator pressure needs to drop before the shells will start moving again.

Although Fig. 12 shows this control approach in relation to a measurement sequence in which pressure is decreasing (e.g., a deflation cycle), the same approach can be applied to a measurement sequence in which pressure is increasing (e.g., an inflation cycle).

Preferably, the sliding interface between the overlapping surfaces of the shell structure has a low dynamic and static friction coefficient. For example, preferably the dynamic friction coefficient is below 0.2 and more preferably below 0.09-0.14. Furthermore, preferably the difference between the dynamic and static friction coefficient is less than 0.05 and more preferably less than 0.03.

Embodiments of the invention described above employ a controller unit. The controller unit may comprise one or more processors. It may be located in a single containing device, structure or unit, or it may be distributed between a plurality of different devices, structures or units. Reference therefore to the controller unit being adapted or configured to perform a particular step or task may correspond to that step or task being performed by any one or more of a plurality of controller components, either alone or in combination. The skilled person will understand how such a distributed processing device can be implemented. The controller unit may include a communication module or input/output for receiving data and outputting data to further components.

The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (30) for use in hemodynamic parameter measurement comprising:
a cuff for extending around a body part of a user, and wherein the cuff includes a pressure actuator (54) operable to apply a variable pressure to the body part by the cuff,
a tissue pressure sensor (52) arranged for sensing a pressure between a surface of the body part and the cuff;
a shell structure (12) for extending around the body part and for arrangement between the tissue pressure sensor and the pressure actuator during operation, the shell structure having at least one pair of tangentially overlapping surfaces which are tangentially slidable relative to one another to permit variation in a diameter of the shell structure; and
a controller unit (32), adapted to:
control a measurement cycle for the cuff which comprises controlling the pressure actuator to progress through a sequence of discrete applied pressure values, wherein each applied pressure value is held constant for an application period; and
record a respective tissue pressure signal from the tissue pressure sensor during each application period.

2. The system of claim 1, wherein a transition time between each pair of consecutive discrete applied pressure values is shorter than any application period.

3. The system of claim 1 or 2, wherein the discrete sequence of applied pressure values is pre-determined, and optionally wherein the applied pressure values are spaced at regular pressure intervals.

4. The system of any of claims 1-3, wherein at least a subset of the applied pressure values of the sequence of applied pressure values are determined in real time during the measurement cycle based on acquired tissue pressure measurements.

5. The system of claim 4, wherein, for at least a subset of the sequence of applied pressure values, a next applied pressure value in the sequence is determined based on a tissue pressure measurement obtained at a current or previous applied pressure value of the sequence.

6. The system of claim 4 or 5, wherein a transition between at least one pair of applied pressure levels in the sequence comprises:
identifying or determining a target next applied pressure level in the sequence;
transitioning the actuator applied pressure to the target applied pressure level while simultaneously sampling and recording the tissue pressure sensor signal;
extracting a baseline trend from the tissue pressure signal over a period spanning the transition;
determining an expected change in the baseline trend in view of the change in applied pressure, and comparing the actual change in the baseline trend over the transition period with the expected change;
wherein, if the actual change is lower than the expected change by a pre-determined threshold amount, the applied pressure is incrementally adjusted in a direction toward a next applied pressure value in the sequence, until the baseline trend rises or drops to the level expected, given the total change in applied pressure.

7. The system of any of claims 1-6, wherein the controller unit is adapted to monitor an amplitude of the tissue pressure signal recorded at each applied pressure, detect occurrence of a peak in the amplitude of the tissue pressure signal, determine a termination pressure for the measurement cycle based on the applied pressure value coincident with the detected peak amplitude, and terminate the measurement cycle at said determined termination pressure.

8. The system of any of claims 1-7, wherein each application period spans at least 1-3 heart pulses.

9. The system of claim 8, wherein each application period spans a single heart cycle.

10. The system of any of claims 1-9, wherein the cuff further comprises a releasable securement means for securing the wrapped cuff around the arm to prevent unwrapping, and wherein the securement means comprises a hook and loop type coupling.

11. A method for controlling a measurement cycle of a hemodynamic parameter measurement apparatus,
wherein the apparatus comprises:
a cuff for wrapping around a body part of a user, and wherein the cuff includes a pressure actuator operable to apply a variable pressure to the body part by the cuff,
a tissue pressure sensor arranged for sensing a pressure between a surface of the body part and the cuff, and
a shell structure for extending around the body part and for arrangement between the tissue pressure sensor and the pressure actuator during operation, the shell structure having at least one pair of tangentially overlapping surfaces which are tangentially slidable relative to one another to permit variation in a diameter of the shell structure; and
the method comprising:
controlling a measurement cycle for the cuff which comprises controlling the pressure actuator to progress through a sequence of discrete applied pressure values; and
recording a respective tissue pressure signal from the tissue pressure sensor during each application period.

12. The method of claim 11, wherein a transition time between each pair of consecutive discrete pressure values is shorter than any application period.

13. The method of claim 11 or 12, further comprising monitoring an amplitude of the tissue pressure signal recorded at each applied pressure, detecting occurrence of a peak in the amplitude of the tissue pressure signal, determining a termination pressure for the measurement cycle based on the applied pressure value coincident with the detected peak amplitude, and terminating the measurement cycle at said determined termination pressure.

14. A computer program product comprising code means configured, when run on a processor operatively coupled with a hemodynamic parameter measurement apparatus as recited in claim 11, to cause the processor to perform a method in accordance with any of claims 11-13.
